# EUROPEAN PATENT SPECIFICATION

(11) **EP 4 088 757 B1**
(45) Date of publication and mention of the grant of the patent: **10.12.2025**
(21) Application number: 21753948.5
(22) Date of filing: 15.02.2021
(51) Int. Cl.: A61L 27/52, A61L 27/56, A61L 27/54, C08B 37/08, C08J 3/075, A61L 27/38, A61L 27/28, C08B 37/00

(54) **PHENOL DERIVATIVE-FUNCTIONALIZED CHONDROITIN SULFATE HYDROGEL AND USE OF SAME**
PHENOLDERIVAT-FUNKTIONALISIERTES CHONDROITINSULFAT-HYDROGEL UND SEINE VERWENDUNG
HYDROGEL DE SULFATE DE CHONDROÏTINE FONCTIONNALISÉ PAR UN DÉRIVÉ DE PHÉNOL ET SON UTILISATION

(30) Priority: 14.02.2020 KR 20200018358; 25.01.2021 KR 20210010484
(43) Date of publication of application: 16.11.2022
(73) Proprietor: Cellartgen Inc., Seoul 03722 (KR)
(72) Inventor: CHO, Seung Woo, Seoul 03722 (KR); YUN, In Sik, Seoul 06273 (KR); SHIN, Ji Soo, Seoul 03722 (KR); CHOI, Soo Jeong, Seoul 03722 (KR)
(74) Representative: Manitz Finsterwald Patent- und Rechtsanwaltspartnerschaft mbB
(86) International application number: PCT/KR2021/001923
(87) International publication number: WO 2021/162529

(56) References cited:
- EP-A1- 3 981 393
- WO-A1-2018/143736
- WO-A1-2021/006426
- JP-A- 2014 514 942
- KR-A- 20190 052 547
- KR-B1- 102 071 029
- US-B2- 8 137 688
- RONG JIN ET AL: "Tyrosinase-mediated in situ forming hydrogels from biodegradable chondroitin sulfate-tyramine conjugates", POLYMER INTERNATIONAL, vol. 62, no. 3, 29 June 2012 (2012-06-29), GB, pages 353 - 361, XP055300486, ISSN: 0959-8103, DOI: 10.1002/pi.4306
- HAN LU ET AL: "Mussel-Inspired Tissue-Adhesive Hydrogel Based on the Polydopamine-Chondroitin Sulfate Complex for Growth-Factor-Free Cartilage Regeneration", APPLIED MATERIALS & INTERFACES, vol. 10, no. 33, 27 July 2018 (2018-07-27), US, pages 28015 - 28026, XP093109399, ISSN: 1944-8244, DOI: 10.1021/acsami.8b05314
- ZHU WENZHEN ET AL: "A DOPA-functionalized chondroitin sulfate-based adhesive hydrogel as a promising multi-functional bioadhesive", JOURNAL OF MATERIALS CHEMISTRY. B, vol. 7, no. 10, 1 January 2019 (2019-01-01), GB, pages 1741 - 1752, XP093109410, ISSN: 2050-750X, DOI: 10.1039/C8TB01990H
- CHOI ILNAM, KIM CHANGHYUN, SONG JEONG EUN, BAEK JONGSEON, JEON SUNG HYUN, JEON HAYAN, LEE SOON YOUNG, KHANG GILSON: "A Comprehensive Study on Cartilage Regeneration Using Gellan-gum/Chondroitin Sulfate Hybrid Hydrogels", PORRIME - POLYMER, NGUG GOBUNJA HAGHOI, SEOUL,, HA, vol. 41, no. 6, 30 November 2017 (2017-11-30), HA, pages 962 - 966, XP055835957, ISSN: 0379-153X, DOI: 10.7317/pk.2017.41.6.962

## Description

### TECHNICAL FIELD

The present invention relates to a hydrogel of chondroitin sulfate functionalized with a phenol derivative, to said hydrogel for various uses and to methods for preparing said hydrogel, and is defined by the appended claims.

### BACKGROUND

Currently, many surgical operations, such as microfracture, autologous cartilage tissue implantation and chondrocyte and stem cell implantation, and cell therapies are being conducted to treat damaged cartilage tissues. Microfracture can induce regeneration of fibrous cartilage and thus cannot be a fundamental therapy. In the case of autologous cartilage tissue implantation and chondrocyte and stem cell implantation, implanted cells and tissues cannot be maintained at a damaged site and may be carried away or annihilated by the surrounding environment at a site of lesion such as inflammation. Thus, their therapeutic effect is insignificant.

Therefore, various tissue engineering methods have been developed to overcome the limitations of these surgical operations and cell therapy products, but there is still no effective system to be used in actual clinical treatment. In particular, although a biocompatible hydrogel containing an extracellular matrix, such as collagen, hyaluronic acid and chondroitin sulfate, as a main component has been developed as a three-dimensional scaffold for effective autologous cartilage tissue implantation, cell implantation and drug delivery. However, there have been limitations in inducing cartilage regeneration over a long period of time due to difference in mechanical properties from the actual cartilage tissue, degradation behavior which is difficult to control and low engraftment rate caused by a weak tissue adhesive property. The following is knwon from the prior art: in Polymer International, 2012, vol. 62, pages 353-361 a hydrogel comprising chondroitin sulfate and tyramine is disclosed; in Journal of Materials Chemistry B, 2019, vol. 7, pages 1741-1752 a hydrogel comprising chondroitin sulfate and dopamine is disclosed.

The present disclosure relates to development of a tissue adhesive hydrogel based on a chondroitin sulfate derivative functionalized with a phenol derivative and its use as a therapeutic agent for cartilage regeneration. A hydrogel was prepared by introducing a catechol group derived from a mussel's adhesive substance or a gallol group derived from a ascidian's adhesive substance into a chondroitin sulfate polymer abundant in the cartilage tissue in the body. The developed hydrogel can promote differentiation of three-dimensionally cultured stem cells into chondrocytes by providing a microenvironment similar to the cartilage tissue, and can be effectively attached to a damaged cartilage site with an excellent tissue adhesive property to promote cartilage regeneration. The tissue adhesive chondroitin hydrogel can be applied to sustained drug delivery as well as cell therapies and autologous cartilage tissue implantation, and thus can induce cartilage regeneration in various ways.

In particular, unlike a case where an oxidizer is used for cross-linking in a chondroitin sulfate hydrogel system functionalized with a catechol group, a chondroitin sulfate hydrogel functionalized with a gallol group, which has more excellent oxidativity, can be crosslinked through natural oxidation without an additional treatment of an oxidizer and thus is improved in stability. Thus, its use can be further expanded.

### DISCLOSURE OF THE INVENTION

### PROBLEMS TO BE SOLVED BY THE INVENTION

The present disclosure is conceived to provide a hydrogel including chondroitin sulfate functionalized with a phenol derivative.

The present disclosure is also conceived to provide a composition for cell culture including the hydrogel.

The present disclosure is also conceived to provide a composition for promoting cell differentiation including the hydrogel.

The present disclosure is also conceived to provide a composition for drug delivery including the hydrogel.

The present disclosure is also conceived to provide a composition for promoting tissue regeneration including the hydrogel.

The present disclosure is also conceived to provide a composition for tissue implantation including the hydrogel.

The present disclosure is also conceived to provide a method of preparing a hydrogel based on chondroitin sulfate functionalized with a phenol derivative, including a process of functionalizing chondroitin sulfate with a phenol derivative.

### MEANS FOR SOLVING THE PROBLEMS

In order to solve the above-described problems, the present disclosure provides a hydrogel including chondroitin sulfate functionalized with a phenol derivative, wherein the hydrogel has a structure represented by Chemical Formula 1: 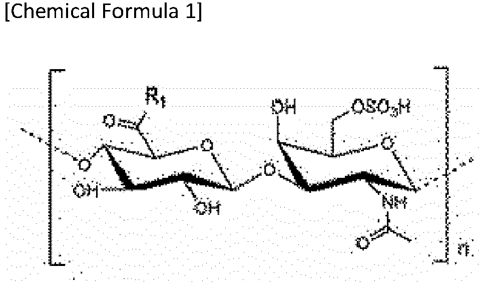 wherein in Chemical Formula 1, R¹ is a phenol derivative, wherein the phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

Further, the present disclosure provides a composition for cell culture including the hydrogel.

Furthermore, the present disclosure provides a composition for promoting cell differentiation including the hydrogel.

Moreover, the present disclosure provides a composition for drug delivery including the hydrogel.

Also, the present disclosure provides a composition for promoting tissue regeneration including the hydrogel.

Further, the present disclosure provides a composition for tissue implantation including the hydrogel.

Furthermore, the present disclosure provides a method of preparing a hydrogel based on chondroitin sulfate functionalized with a phenol derivative, including a process of functionalizing chondroitin sulfate with a phenol derivative, wherein the hydrogel has a structure represented by Chemical Formula 1: 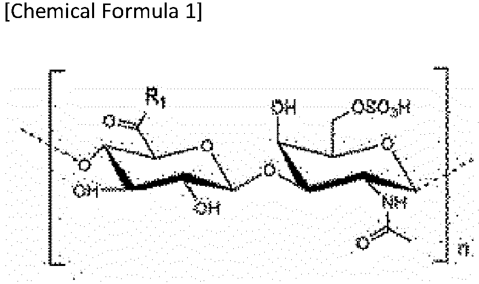 wherein in Chemical Formula 1, R¹ is a phenol derivative, wherein the phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

### EFFECTS OF THE INVENTION

Due to the population aging and the increase in daily sports, such as sports and mountain climbing, and leisure activities, the incidence of cartilage damage and related diseases is rapidly increasing worldwide. The market size for diseases related to cartilage loss, such as cartilage arthritis, is expected to increase from $4.3 billion in 2017 to $9.6 billion in 2024. While these values seem large, the market size is estimated small compared to the number of patients because there is no fundamental therapeutic agent. Currently, drug therapy or surgery has been used mainly. However, the number of patients who want cartilage replacement to improve their quality of life has increased by more than 80% over the past 5 years, and has shown an increase rate of more than 30% every year. Considering the huge marketability of cartilage regeneration technology, it is expected that a three-dimensional biomaterial scaffold-based therapeutic agent with an enhanced cartilage regeneration ability will be able to create enormous added value when it is successfully commercialized.

According to the present disclosure, in order to overcome the limitations of the prior art, a gallol group derived from the adhesive of mussels or sea squirts is introduced into a chondroitin sulfate polymer, which is an extracellular matrix abundant in the cartilage tissue, to provide an environment similar to the cartilage tissue and also facilitate regulation of mechanical properties and degradation rate and greatly improve a tissue adhesive property. Therefore, cell, tissue, drug delivery efficiency and cartilage regeneration efficiency are improved. The hydrogel of the present disclosure has mechanical, physiochemical and biomechanical properties suitable for cartilage regeneration and also has excellent biocompatibility and tissue adhesive property. Therefore, it shows high potential as a cell, tissue and drug delivery platform for cartilage regeneration treatment at a clinically applicable level.

A biomimetic adhesive chondroitin sulfate hydrogel system functionalized with a phenol derivative according to the present disclosure provides a mechanical and biochemical microenvironment similar to the cartilage tissue and has an excellent tissue adhesive property and thus can overcome the limitations of conventional cell, tissue and drug therapies. Therefore, it is expected to be developed as a new concept medical preparation that can induce long-term cartilage regeneration, which has not been realized yet. Since there is currently no fundamental therapeutic agent for cartilage tissue regeneration at home and abroad, its marketability is expected to be very large. Also, it is expected to replace foreign functional biomaterials and tissue regeneration technologies as well as domestic ones and thus achieve a huge import substitution effect.

The developed hydrogel can be applied to various types of formulations such as particles, beads and patches as well as injectable formulations. Thus, it can be applied to various tissue injuries as well as cartilage regeneration. Therefore, its clinical application range can be greatly expanded.

### BRIEF DESCRIPTION OF THE DRAWINGS

**FIG.** 1A is a schematic diagram showing a synthesis process of a hydrogel (CS-CA hydrogel) based on chondroitin sulfate functionalized with catechol, and **FIG. 1B** is a ¹H-NMR analysis result of the CS-CA hydrogel.
**FIG. 2A** shows the molecular ratios between reactants during a preparation process of the CS-CA hydrogel and the resultant degrees of catechol substitution, and **FIG. 2B** shows images before and after gelation.
**FIG. 3A** shows an analysis result of swelling behavior of the CS-CA hydrogel depending on the the degree of substitution of catechol and the concentration of CS-CA of catechol.
**FIG. 3B** shows an analysis result of swelling behavior of the CS-CA hydrogel depending on the the degree of substitution of catechol and the concentration of CS-CA of catechol.
**FIG. 3C** shows an analysis result of a degradation behavior of the CS-CA hydrogel.
**FIG. 4** shows an analysis result of mechanical properties (FIG. 4A: elastic modulus; and FIG. 4B: compressive modulus) of the CS-CA hydrogel depending on the degree and substitution and the concentration of catechol.
**FIG. 5** shows a measurement result of tissue adhesive strength (FIG. 5A: method; FIG. 5B: adhesive strength; and FIG. 5C: work of adhesion) of the CS-CA hydrogel depending on the degree and substitution and the concentration of catechol (control group: CS-ME (methacrylated CS) hydrogel).
**FIG. 6A** shows a measurement result of a cell viability after three-dimensional culture of human adipose-derived stem cells in the CS-CA hydrogel depending on the degree and substitution and the concentration of catechol (control group: CS-ME hydrogel).
**FIG. 6B** shows a measurement result of a cell viability after three-dimensional culture of human adipose-derived stem cells in the CS-CA hydrogel depending on the degree and substitution and the concentration of catechol (control group: CS-ME hydrogel).
**FIG. 7A** shows a measurement result of the amount of pro-inflammatory cytokine TNF-α (tumor necrosis factor-a) secreted from macrophages after indirect co-culture of the CS-CA hydrogel and the macrophages.
**FIG. 7B** shows a measurement result of the amount of pro-inflammatory cytokine TNF-α (tumor necrosis factor-a) secreted from macrophages after direct co-culture of the CS-CA hydrogel and the macrophages.

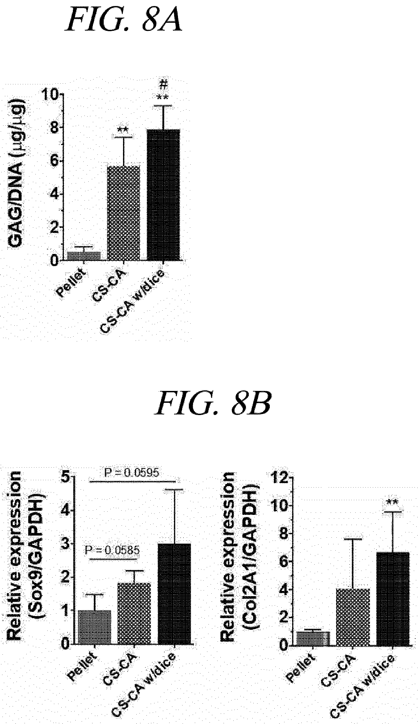

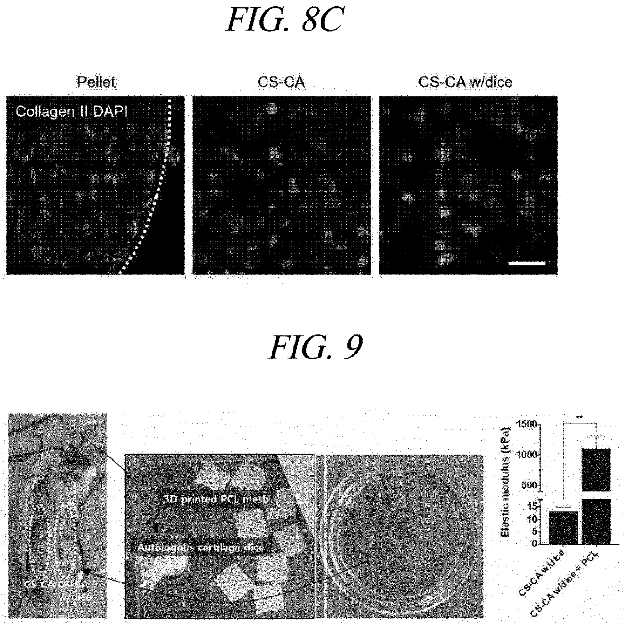

**FIG. 8A** shows a measurement result of the amount of glycosaminoglycan (GAG), which is a cartilage differentiation index, after three-dimensional culture of human adipose-derived stem cells (hADSC) in the CS-CA hydrogel and differentiation in a cartilage differentiation-inducing culture medium, **FIG. 8B** shows a measurement result of the expression levels of cartilage differentiation markers SOX9 and collagen type II (col2A1) through real-time qPCR, and **FIG. 8C** shows a measurement result of the expression level of collagen type II through immunofluorescence staining (Pellet: a conventional culture method for culturing and differentiating cells the form of pellets; CS-CA: a method for encapsulating stem cells in the CS-CA hydrogel for three-dimensional culture and differentiation; and CS-CA w/dice: a method for encapsulating stem cells with human-derived decellularized cartilage tissue dice in the CS-CA hydrogel for culture and differentiation in a three-dimensional environment similar to the actual cartilage tissue).
**FIG. 9A** shows a method of manufacturing a graft by extracting cartilage from a rabbit's ear, mixing it with the CS-CA hydrogel, and applying the mixture to a PCL scaffold, and **FIG. 9B** shows an analysis result of mechanical properties (elastic modulus).

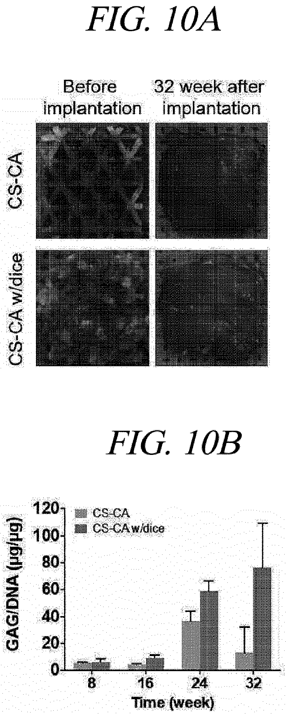

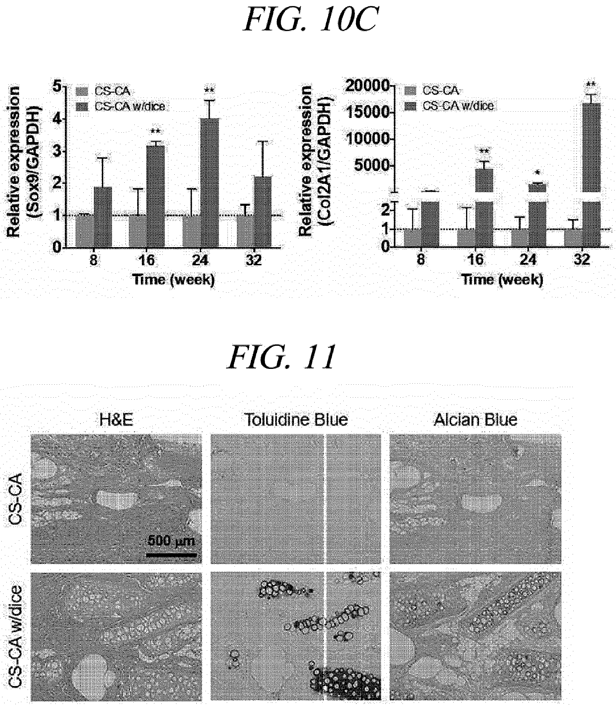

**FIG. 10A** shows a comparison result between the manufactured graft before implantation and the retrieved graft 32 weeks after implantation, **FIG. 10B** shows a measurement result of the amount of glycosaminoglycan (GAG), which is a cartilage differentiation index, **FIG. 10C** shows a measurement result of the expression levels of cartilage differentiation markers SOX9 and collagen type II (col2A1) through real-time qPCR (CS-CA: a group in which only the CS-CA hydrogel is implanted into a PCL scaffold; and CS-CA w/dice: a group in which a CS-CA hydrogel including autologous cartilage tissue dice is implanted into a PCL scaffold).
**FIG. 11** shows a result of histological analysis conducted 32 weeks after implantation of the autologous cartilage tissue (CS-CA: a group in which only the CS-CA hydrogel is implanted into a PCL scaffold; and CS-CA w/dice: a group in which a CS-CA hydrogel including autologous cartilage tissue dice is implanted into a PCL scaffold).
**FIG. 12A** shows a result of evaluation on the efficiency of cartilage tissue implantation and the efficiency of tissue integration after implantation of a rabbit's ear cartilage tissue into an mouse model of auricular cartilage defect by using the CS-CA hydrogel or a conventional photo-crosslinked CS-ME hydrogel (CS-ME w/dice: a group in which a CS-ME hydrogel including cartilage tissue (dice) is implanted; and CS-CA w/dice: a group in which the CS-CA hydrogel including cartilage tissue (dice) is implanted).
**FIG. 12B** shows a result of evaluation on the efficiency of cartilage tissue implantation and the efficiency of tissue integration after implantation of a rabbit's ear cartilage tissue into an ear cartilage damage mouse model by using the CS-CA hydrogel or a conventional photo-crosslinked CS-ME hydrogel (CS-ME w/dice: a group in which a CS-ME hydrogel including cartilage tissue (dice) is implanted; and CS-CA w/dice: a group in which the CS-CA hydrogel including cartilage tissue (dice) is implanted).
**FIG. 13** shows a result of checking a drug release profile depending on the presence or absence of treatment with a chondroitinase after TGF-β is encapsulated in the CS-CA hydrogel.
**FIG. 14** shows a result of checking a drug release profile depending on the presence or absence of treatment with a chondroitinase after TGF-β is encapsulated in the CS-CA hydrogel patch (Patch (CS-CA)-TGF-β: a group prepared by encapsulating TGF-β protein in the CA hydrogel patch; and Patch (CS-CA)-TGF-β/FD: a group prepared by previously mixing TGF-β protein with a CS-CA solution, lyophilizing the mixture in the form of a patch and crosslinking the CS-CA hydrogel patch).
**FIG. 15A** is a schematic diagram showing a synthesis process of a hydrogel (CS-PG hydrogel) based on chondroitin sulfate functionalized with pyrogallol, and **FIG. 15B** is a ¹H-NMR analysis result of the CS-PG hydrogel.
**FIG. 16A** shows images of the CS-PG hydrogel before and after gelation, **FIG. 16B** and **FIG. 16C** show an analysis result of swelling behavior of the CS-PG hydrogel depending at two concentrations (10 wt%, 15 wt%), and **FIG. 16D** shows an analysis result of a degradation behavior of the CS-PG hydrogel.
**FIG. 17** shows an analysis result of an elastic modulus depending on the frequency (0.1 Hz to 10 Hz) of the CS-CA hydrogel at two concentrations (10 wt%, 15 wt%) **(****FIG. 17A****)** and an analysis result of an average elastic modulus at a frequency of 1 Hz **(****FIG. 17B****).**
**FIG. 18** shows a measurement result of a cell viability after three-dimensional culture of human adipose-derived stem cells in the CS-PG hydrogel.

### BEST MODE FOR CARRYING OUT THE INVENTION

The present disclosure relates to a hydrogel including chondroitin sulfate functionalized with a phenol derivative.

In the present disclosure, the hydrogel has a structure represented by Chemical Formula 1: 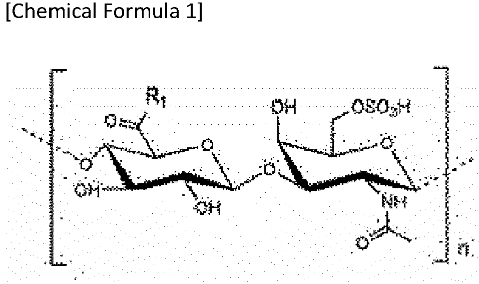 in Chemical Formula 1, R₁ is a phenol derivative.

In the present disclosure, the phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

In an embodiment of the present disclosure, the hydrogel may be prepared through oxidation of the functionalized phenol derivative.

In an embodiment of the present disclosure, the oxidation may be carried out by addition of an oxidizer or an enzyme.

In an embodiment of the present disclosure (outside the scope of the claims), the oxidation may be carried out by a body enzyme without addition of an oxidizer or an enzyme.

In an embodiment of the present disclosure, the hydrogel may have an elastic modulus of from 0.1 kPa to 100 kPa at from 0.1 Hz to 10 Hz.

In an embodiment of the present disclosure, the hydrogel may have a tissue adhesive property.

In an embodiment of the present disclosure, the hydrogel may be biodegradable.

Further, the present disclosure provides a composition for cell culture including the hydrogel.

In an embodiment of the present disclosure, the cell culture may be a three-dimensional culture.

Furthermore, the present disclosure provides a composition for promoting cell differentiation including the hydrogel.

In an embodiment of the present disclosure, the cell may be a stem cell.

In an embodiment of the present disclosure, the stem cell may be selected from the group consisting of embryonic stem cells, fetal stem cells, induced pluripotent stem cells and adult stem cells.

In an embodiment of the present disclosure, the composition may promote differentiation into chondrocytes.

Moreover, the present disclosure provides a composition for drug delivery including the hydrogel.

In an embodiment of the present disclosure, the drug may be encapsulated or loaded in the hydrogel.

In an embodiment of the present disclosure, the drug may be selected from the group consisting of an immune cell activator, an anticancer agent, a therapeutic antibody, an antibiotic, an antibacterial agent, an antiviral agent, an anti-inflammatory agent, a contrast medium, a protein drug, a growth factor, a cytokine, a peptide drug, a hair growth solution, an anesthetic and combinations thereof.

In an embodiment of the present disclosure, the composition may be in the form of an adhesive patch or a film.

Also, the present disclosure provides a composition for promoting tissue regeneration including the hydrogel.

Further, the present disclosure provides a composition for tissue implantation including the hydrogel.

In an embodiment of the present disclosure, the compositions may be in the form of an adhesive patch or a film.

In an embodiment of the present disclosure, the tissue may be selected from the group consisting of liver, heart, kidney, muscle, stomach, intestine, lung, bone, cartilage, blood vessel, bladder, skin, brain, fat, thyroid gland, salivary gland, oesophagus, pancreas, spinal cord, ligament, tendon, tooth and uterus.

Furthermore, the present disclosure provides a method of preparing a hydrogel based on chondroitin sulfate functionalized with a phenol derivative, including a process of functionalizing chondroitin sulfate with a phenol derivative. The hydrogel has a structure represented by Chemical Formula 1: 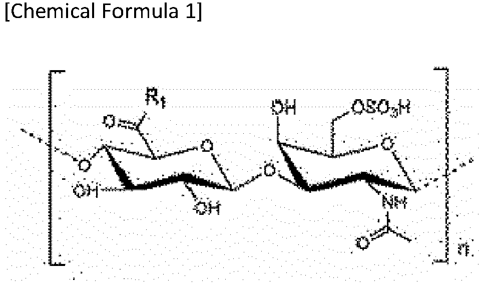 In Chemical Formula 1, R¹ is a phenol derivative. The phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

In an embodiment of the present disclosure, the method may further include a process of oxidizing the functionalized phenol derivative.

### MODE FOR CARRYING OUT THE INVENTION

The present disclosure provides a hydrogel including chondroitin sulfate functionalized with a phenol derivative.

In the present disclosure, the hydrogel has a structure represented by Chemical Formula 1: 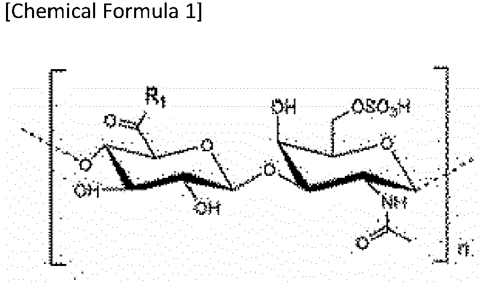 in Chemical Formula 1, R₁ is a phenol derivative.

In the present disclosure, the phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol, but is not limited thereto.

In an example of the present disclosure, in chondroitin sulfate functionalized with a phenol derivative, the hydrogel may be crosslinked through oxidation of the functionalized phenol derivative. The oxidation may be carried out by a body enzyme without addition of an oxidizer or an enzyme. The oxidizer may be sodium periodate (NaIO₄) and hydrogen peroxide (H₂O₂), and the enzyme may be a peroxidase, but is not limited thereto.

Also, the oxidation may be carried out by a body enzyme without addition of an oxidizer or an enzyme. When the hydrogel is functionalized with a phenol derivative with stronger oxidativity, for example, pyrogallol, it may be cross-liked through oxidation with a body enzyme without using an additional oxidizer or enzyme.

In an example of the present disclosure, the hydrogel may have an elastic modulus of from 0.1 kPa to 100 kPa at from 0.1 Hz to 10 Hz. Desirably, the hydrogel may have an elastic modulus of from 0.1 kPa to 50 kPa, from 0.1 kPa to 30 kPa, from 0.1 kPa to 20 kPa, from 0.1 kPa to 10 kPa and from 1 kPa to 10 kPa, but is not limited thereto.

In an example of the present disclosure, the hydrogel may have a tissue adhesive property or may be biodegradable.

Further, the present disclosure provides a composition for cell culture including a hydrogel based on chondroitin sulfate functionalized with a phenol derivative.

In an example of the present disclosure, the culture may be a three-dimensional culture. The cells may be stem cells, hematopoietic stem cells, hepatic cells, fibrous cells, epithelial cells, mesothelial cells, endothelial cells, muscle cells, nerve cells, immune cells, adipocytes, chondrocytes, bone cells, blood cells or skin cells, and can be applied to all cells capable of growth in the hydrogel of the present disclosure regardless of the cell type.

Furthermore, the present disclosure provides a composition for promoting cell differentiation including a hydrogel based on chondroitin sulfate functionalized with a phenol derivative.

In an example of the present disclosure, the cell may be a stem cell, and the stem cell may be selected from the group consisting of embryonic stem cells, fetal stem cells, induced pluripotent stem cells and adult stem cells.

In an example of the present disclosure, the composition may promote differentiation from the stem cell into chondrocytes.

Moreover, the present disclosure provides a composition for drug delivery including a hydrogel based on chondroitin sulfate functionalized with a phenol derivative.

In an example of the present disclosure, the drug may be encapsulated or loaded in the hydrogel, and may be selected from the group consisting of an immune cell activator, an anticancer agent, a therapeutic antibody, an antibiotic, an antibacterial agent, an antiviral agent, an anti-inflammatory agent, a contrast agent, a protein drug, a growth factor, a cytokine, a peptide drug, a hair growth agent, an anesthetic and combinations thereof, but is not limited thereto.

The anticancer agent may be selected from the group consisting of anthracyclines such as doxorubicin, daunomycin, epirubicin, idarubicin, etc.; taxanes such as paclitaxel, docetaxel, cabazitaxel, tesetaxel, etc.; alkaloids such as curcumin, camptothecin, berberine, evodiamine, matrine, piperine, sanguinarine, tetrandrine, thalicarpine, ellipticine, etc.; vinca alkaloids vinblastine, vincristine, vindesine, vinorelbine, etc.; platinums such as cisplatin, carboplatin, oxaliplatin, etc.; antimetabolites such as 5-fluorouracil, capecitabine, methotrexate, gemcitabine, etc.; topoisomerase inhibitors such as irinotecan, topotecan, etoposide, teniposide, etoposide, amsacrine, etc.; antitumor antibiotics such as bleomycin, actinomycin, mitomycin, mitoxantrone, etc.; alkylating agents such as cyclophosphamide, mechlorethamine, chlorambucil, melphalan, nitrosourea, etc.; nucleoside analogs such as azacytidine, azathioprine, cytarabine, doxifluridine, hydroxyurea, mercaptopurine, methotrexate, etc.; genetic drugs such as small interfering RNA (siRNA), small hairpin RNA (shRNA), microRNA (miRNA), plasmid DNA, etc.; enzymes such as L-asparaginase, etc.; hormones such as triptorelin acetate, megestrol acetate, flutamide, bicalutamide, goserelin, etc.; cytochrome C; and p53 protein, but is not limited thereto.

The therapeutic antibody may be selected from the group consisting of trastuzumab, rituximab, bevacizumab, cetuximab, bortezomib, erlotinib, gefitinib, imatinib mesylate, sunitinib, pembrolizumab, nivolumab, atezolizumab, ipilimumab and blinatumomab, but is not limited thereto.

The antibiotic may be selected from the group consisting of β-lactams, aminoglycosides, macrolides, tetracyclines, glycopeptides, lincosamides, quinolones, chloramphenicol, sulfa drugs, trimethoprim, polymyxin, bacitracin, mupirocin, fusidic acid, streptogramin and oxazolidinone, but is not limited thereto.

The antibacterial agent may be selected from the group consisting of metronidazole, secnidazole, ornidazole, tinidazole, clindamycin, sodium polystyrene sulfate and sodium cellulose sulfate, but is not limited thereto.

The antiviral agent may be selected from the group consisting of acyclobyl, vidarabine, ganciclovir, foscarnet, famciclovir, ribavirin, amantadine, zanamivir and oseltamivir, but is not limited thereto.

The anti-inflammatory agent may be selected from the group consisting of NSAIDs such as ibuprofen, dexibuprofen, naproxen, fenoprofen, ketoprofen, dexketoprofen, flurbiprofen, oxaprozin, loxoprofen, indomethacin, tolmetin, sulindac, etodolac, ketorolac, diclofenac, nabumetone, piroxicam, meloxicam, tenoxicam, droxicam, lornoxicam, isoxicam, mefenamic acid, meclofenamic acid, flufenamic acid, tolfenamic acid, niflumic acid, aspirin, diflunisal, salsalate and dexamethasone, but is not limited thereto.

The contrast agent may be selected from the group consisting of a PET contrast agent, a CT contrast agent, an MRI contrast agent, an optical image contrast agent and a US contrast agent, but is not limited thereto.

The growth factor may be selected from the group consisting of a vascular endothelial growth factor, an epidermal growth factor, a keratinocyte growth factor, a growth and differentiation factor, a hepatocyte growth factor, a platelet-derived growth factor, a transforming growth factor, angiopoietin, erythropoietin, a bone morphogenetic protein, an insulin-like growth factor, an acidic and basic fibroblast growth factor, a granulocyte-macrophage colony-stimulating factor, a brain-derived neurotrophic factor, a glial cell-derived neurotrophic factor, a nerve growth factor, a stromal cell-derived factor-1, a substance P and a hypoxia-inducible factor-1, but is not limited thereto.

The cytokine may be selected from the group consisting of interleukin, lymphokine, monokine, chemokine, interferon and adipokine, but is not limited thereto.

The hair growth agent may be selected from the group consisting of finasteride or dutasteride, but is not limited thereto.

The anesthetic may be selected from the group consisting of ropivacaine, bupivacaine, chloroprocaine, lidocaine, mepivacaine, procaine, tetracaine, levobupivacaine and articaine, but is not limited thereto.

In an example of the present disclosure, the composition may be in the form of an adhesive patch or a film.

Also, the present disclosure provides a composition for promoting tissue regeneration including a hydrogel based on chondroitin sulfate functionalized with a phenol derivative.

Further, the present disclosure provides a composition for tissue implantation including a hydrogel based on chondroitin sulfate functionalized with a phenol derivative.

In an example of the present disclosure, the tissue may be selected from the group consisting of liver, heart, kidney, muscle, stomach, intestine, lung, bone, cartilage, blood vessel, bladder, skin, brain, fat, thyroid gland, salivary gland, oesophagus, pancreas, spinal cord, ligament, tendon, tooth and uterus. Desirably, the tissue may be a cartilage tissue, but is not limited thereto. Furthermore, the composition may be in the form of an adhesive patch or a film, and the drug may be encapsulated or loaded in the hydrogel.

Furthermore, the present disclosure provides a method of preparing a hydrogel based on chondroitin sulfate functionalized with a phenol derivative, including a process of functionalizing chondroitin sulfate with a phenol derivative and a process of oxidizing the functionalized phenol derivative. The oxidation may be carried out by addition of an oxidizer or an enzyme, or may be carried out by a body enzyme without addition of an oxidizer or an enzyme.

The pharmaceutical composition according to the present disclosure may be prepared into a pharmaceutical dosage form by a well-known method in the art, so that an active component of the composition may be provided via a fast, suspended or prolonged release, after being administered into a mammal. When preparing a dosage form, the pharmaceutical composition according to the present disclosure may further contain a pharmaceutically acceptable carrier, to the extent that this carrier does not inhibit a function of the active component.

The pharmaceutically acceptable carrier may include commonly-used carriers, such as pectin, hyaluronic acid, collagen, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil, but is not limited thereto. In addition, the pharmaceutical composition of the present disclosure may further include a diluent or an excipient, such as fillers, weighting agents, bonding agents, wetting agents, disintegrating agents and surfactants, and other pharmaceutically acceptable additives.

The pharmaceutical composition according to the present disclosure may be administered in a pharmaceutically effective amount. The term "pharmaceutically effective amount" refers to an amount sufficient to prevent or treat a disease at a reasonable benefit/risk ratio applicable to a medical treatment. The effective amount of the pharmaceutical composition of the present disclosure may be determined by a person with ordinary skill in the art according to various factors such as a formulation method, a patient's condition and weight, the patient's gender, age and degree of disease, a drug form, an administration route and period, an excretion rate, reaction sensitivity, etc. The effective amount may vary depending on a route of treatment, a use of excipients and a possibility of being used with other drugs, as recognized by a person with ordinary skill in the art.

The pharmaceutical composition of the present disclosure may be administered to mammals such as mice, livestock, humans, etc. through various routes. Specifically, the pharmaceutical composition of the present disclosure can be administered orally or parenterally (for example, applied or injected intravenously, subcutaneously or intraperitoneally). A solid preparation for oral administration may include powder, granule, tablet, capsule, soft capsule, pill, etc. A liquid preparation for oral administration may include suspension, liquid for internal use, emulsion, syrup, aerosol, etc., but may also include various excipients, for example, wetting agent, sweetener, flavoring agent and preservative in addition to generally used simple diluents such as water and liquid paraffin. A preparation for parenteral administration may be used by being formulated into a dosage form of external preparation and sterilized injectable preparation such as sterilized aqueous solution, liquid, water insoluble excipient, suspension, emulsion, eye drop, eye ointment, syrup, suppository and aerosol according to respective conventional methods. Specifically, the pharmaceutical composition may be used in the form of cream, gel, patch, spraying agent, ointment, plaster, lotion, liniment, eye ointment, eye drop, paste, or cataplasma, but is not limited thereto. A preparation for topical administration may be an anhydrous or aqueous form depending on a clinical prescription. As the water insoluble excipient and the suspension, propylene glycol, polyethylene glycol, vegetable oil such as olive oil, and injectable ester like ethyl oleate, etc. may be used. Base materials of the suppository may include witepsol, macrogol, tween 61, cacao butter, laurinum and glycerogelatin.

The present disclosure relates to a method for synthesizing a chondroitin sulfate derivative functionalized with a phenol derivative (a gallol group) and preparing a hydrogel, and relates to a three-dimensional culture and differentiation of stem cells, autologous cartilage tissue implantation and drug delivery. In the present disclosure, it was confirmed that various properties such as a swelling ratio, a degradation rate, mechanical properties and adhesive properties of a hydrogel can be easily regulated by regulating the degree of catechol group functionalization and the polymer concentration of a synthesized chondroitin sulfate derivative. Accordingly, it was confirmed that the derivative can be transformed and utilized for its purpose and use.

The chondroitin sulfate hydrogel functionalized with a pyrogallol group according to the present disclosure is a biocompatible biomaterial that shows little cytotoxicity and does not cause an immune response. It was confirmed that three-dimensional culture of stem cells is possible, and a method for promoting differentiation into chondrocytes by providing an environment similar to the cartilage tissue was developed. It is expected that the chondroitin sulfate hydrogel can be used as a stem cell implantation scaffold for cartilage tissue regeneration.

Further, in the present disclosure, a novel method for cartilage tissue reconstruction by effectively implanting an autologous cartilage tissue in vivo using excellent tissue adhesive strength of the chondroitin sulfate hydrogel functionalized with a gallol group and inducing cartilage regeneration was developed. By stably delivering the autologous cartilage tissue to a cartilage defective site without damage, it is expected that it is possible to propose a tissue engineering therapy that induces more efficient cartilage regeneration by overcoming the problems related to low engraftment that cannot be solved by the conventional implantation method.

Furthermore, a phenol derivative mimicking the adhesive of mussels or sea squirts and functionalized with a chondroitin sulfate polymer has the ability to be bound to various nucleophilic functional groups through oxidation and thus can be utilized as a system for delivering various drugs having these functional groups. Since it was confirmed that a growth factor protein loaded in a chondroitin sulfate hydrogel according to the present disclosure shows a sustained-controlled release behavior in an in vitro environment simulating the conditions for degradation of chondroitin sulfate in vivo, it is highly expected to be developed into an efficient drug delivery system for cartilage regeneration

Hereinafter, the present disclosure will be explained in more detail with reference to Examples. However, the following Examples are exemplified only to explain the present disclosure but not intended to limit the scope of the present disclosure. Examples 1 to 10 are outside the scope of the claims.

### Example 1. Preparation of hydrogel based on chondroitin sulfate functionalized with phenol derivative (catechol)

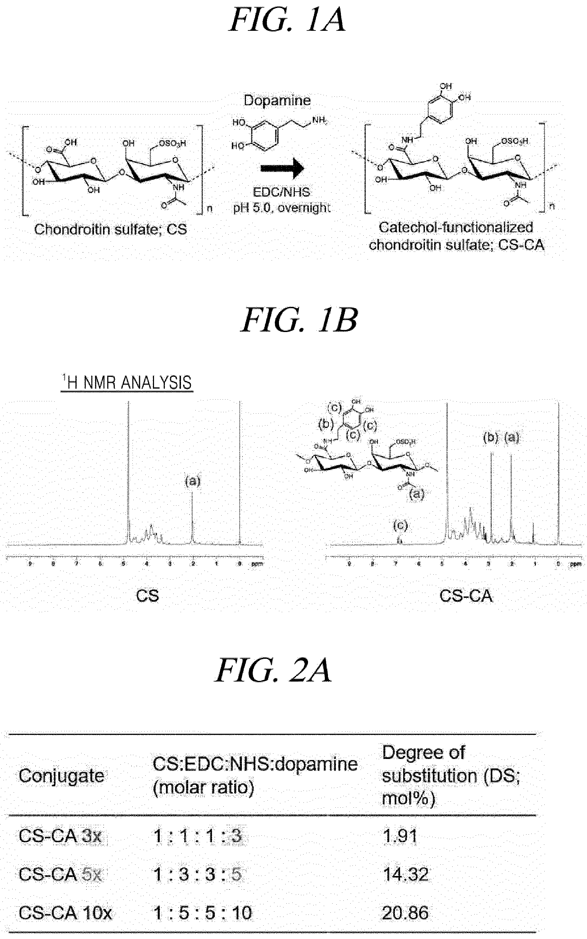

For synthesis of a derivative, chemical reaction between EDC (1-(3-dimethylaminopropyl)-3-ethylcarbodiimide hydrochloride (Thermo Fisher Scientific)) and NHS (N-hydroxysuccinimide (Sigma)) was applied to functionalize chondroitin sulfate (CS) with dopamine **(****FIG. 1A****).** Through ¹H-NMR analysis, it was confirmed from the synthesized derivative that chondroitin sulfate (CS) was successfully functionalized with a catechol group of dopamine **(****FIG. 1B****).**

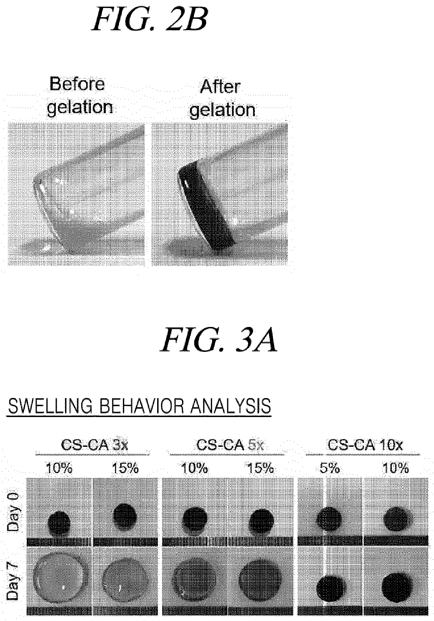

As for the chondroitin sulfate derivative functionalized with catechol (hereinafter, referred to as "CS-CA derivative"), the degree of substitution (DS) was regulated by regulating the molar ratio of the reactants in the synthesis process **(****FIG.** 2A) and three types of CS-CA derivatives (CS-CA 3x; CS-CA 5x; and CS-CA 10x) having different degrees of substitution were synthesized. Thereafter, a CS-CA solution was prepared using PBS and distilled water, and the CS-CA solution was treated with an oxidizer (sodium periodate (NaIO₄)) through a crosslinking reaction to prepare a catechol-functionalized chondroitin sulfate-catechol hydrogel (hereinafter referred to as "CS-CA hydrogel") **(****FIG. 2B****).** The CS-CA solution and the oxidizer (NaIO₄ solution, 12.5 mg/ml) were treated with 3:1 (volume ratio).

### Example 2. Analysis on physicochemical properties of chondroitin sulfate-catechol hydrogel (CS-CA hydrogel) depending on degree of substitution and concentration of catechol

CS-CA hydrogels were prepared with the three types of CS-CA derivatives having different degrees of substitution at two different concentrations (CS-CA 3x: 10 wt%, 15 wt%; CS-CA 5x: 10 wt%, 15 wt%; and CS-CA 10x: 5 wt%, 10 wt%) and tested to compare a swelling ratio and a degradation behavior depending on the degree of substitution and the concentration of catechol. CS-CA 10x at 15wt% has a lower solubility in water and much higher viscosity compared to other conditions. Therefore, it was excluded from the test.

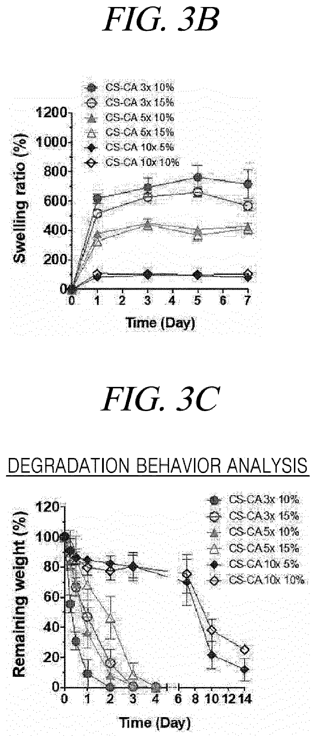

As a result, it was confirmed that as the degree of substitution of catechol decreased, the swelling ratio of the hydrogel increased under constant physiological conditions (37°C in a PBS buffer) for 7 days **(****FIG. 3A** and **FIG. 3B****).** Also, as a result of comparing the degradation behavior through treatment with a chondroitinase, it was confirmed that the degradation of the hydrogel significantly slowed down as the degree of substitution and the concentration of catechol increased **(****FIG. 3C****).**

### Example 3. Analysis on mechanical properties of chondroitin sulfate-catechol hydrogel (CS-CA hydrogel) depending on degree of substitution and concentration of catechol

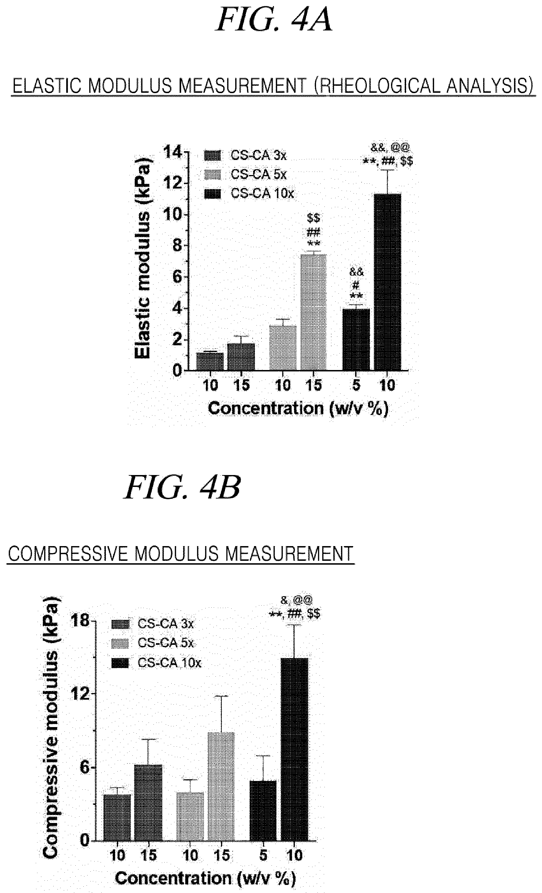

In the present disclosure, a test was conducted to measure the elastic modulus of the CS-CA hydrogel under various degrees of substitution and concentrations of catechol at a frequency of from 0.1 Hz to 10 Hz through rheological analysis. As a result, it was confirmed that as the degree of substitution and the concentration of catechol increased, the elastic modulus increased and the mechanical strength of the hydrogel was improved **(****FIG. 4A****).**

Also, in the present disclosure, a test was conducted to measure the compressive modulus of the CS-CA hydrogel under various degrees of substitution and concentrations of catechol through indentation. As a result, it was confirmed that as the substitution degree and the concentration of catechol increased, the compressive modulus increased like the elastic modulus, which means that the CS-CA hydrogel has sufficient strength to withstand pressure **(****FIG. 4B****).**

Load-bearing force is very important in cartilage regeneration, and it was confirmed from the above results that the CS-CA hydrogel with high degree of substitution and concentration of catechol is suitable for cartilage regeneration.

### Example 4. Measurement of tissue adhesive force of chondroitin sulfate-catechol hydrogel (CS-CA hydrogel) depending on degree of substitution and concentration of catechol

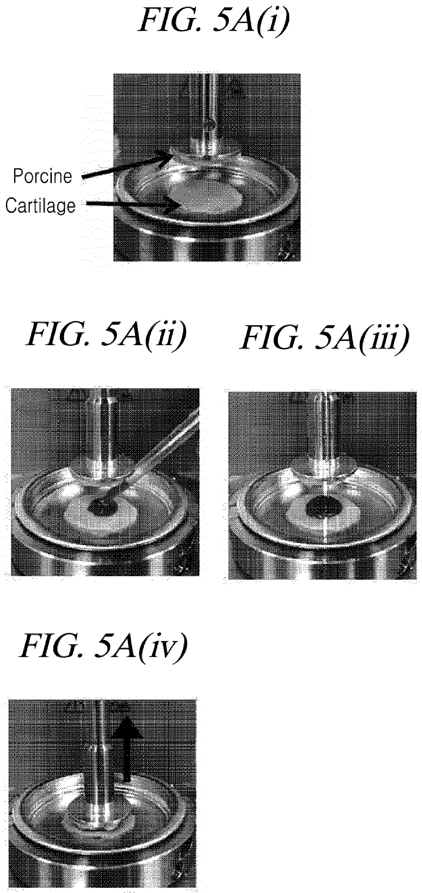

A test was conducted to measure the tissue adhesion force of the hydrogel with a rheometer in tack test mode. In brief, the porcine cartilage was used, and the adhesive strength of CS-CA hydrogels prepared under various degrees of substitution and concentrations of catechol or a CS-ME (methacrylated CS) hydrogel prepared by a conventional photo-crosslinking method were measured and compared **(****FIG. 5A****).**

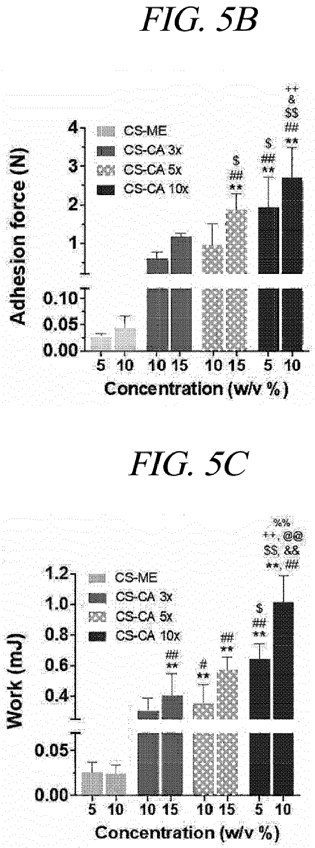

As a result, it was confirmed that the conventional CS-ME hydrogel did not show adhesion force, whereas the CS-CA hydrogels showed excellent tissue adhesive strength under all conditions. Particularly, it was confirmed that the tissue adhesive strength significantly increased in the CS-CA hydrogel with high degree of substitution and concentration of catechol **(****FIG. 5B****).** In addition, it was confirmed that the amount of work (work of adhesion) required to remove the hydrogel attached to the tissue also significantly increased in the CS-CA hydrogel with high degree of substitution and concentration of catechol **(****FIG. 5C****).**

It was confirmed from the above results that all the physicochemical properties, mechanical properties and adhesive strength of the CS-CA hydrogel can be regulated by regulating the degree of substitution and the concentration of catechol.

### Example 5. Analysis on cytotoxicity in three-dimensional stem cell culture using chondroitin sulfate-catechol hydrogel (CS-CA hydrogel)

After three-dimensional culture of human adipose-derived stem cells (hADSC) in the CS-CA hydrogels under various degrees of substitution and concentrations of catechol, a test was conducted to analyze a cell survival rate. In brief, human adipose-derived stem cells were dispensed in each of the CS-CA hydrogels or the CS-ME hydrogel and cultured for 7 days, and then, the cell survival rate was analyzed through live/dead staining.

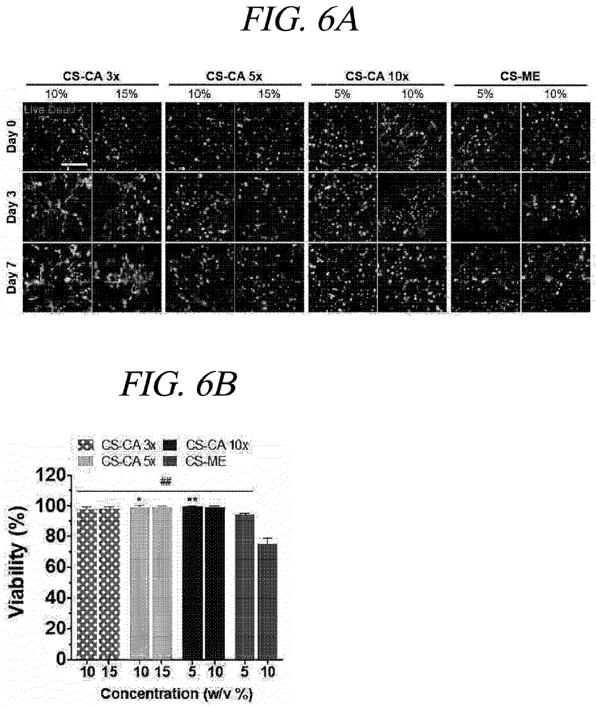

As a result, it was confirmed that the human adipose-derived stem cells cultured in the CS-CA hydrogels under various degrees of substitution and concentrations of catechol showed a high survival rate and thus showed little cytotoxicity **(****FIG. 6A** and **FIG. 6B****).** However, the human adipose-derived stem cells cultured in the conventional CS-ME hydrogel showed a tendency to decrease in cell survival rate as the culture period increased and showed evident cytotoxicity as the concentration increased **(****FIG. 6A** and **FIG. 6B****).**

It was confirmed from the above results that the CS-CA hydrogels do not show cytotoxicity and thus have excellent biocompatibility.

### Example 6. Analysis on immune response of chondroitin sulfate-catechol hydrogel (CS-CA hydrogel)

A test was conducted to check the degree of induction of immune response by measuring the amount of pro-inflammatory cytokine TNF-α (tumor necrosis factor-a) secreted from macrophages through direct/indirect co-culture of a hydrogel and the macrophages. A transwell model was used for indirect co-culture, and an immune response caused by degradation products released from the hydrogel and a material used for crosslinking was checked. In addition, for direct co-culture, a cell culture plate well was pre-coated with a hydrogel and macrophages were dispensed thereon in order for the cells to be directly attached to the hydrogel and cultured, and an immune response caused by direct interaction between the cells and the hydrogel was observed.

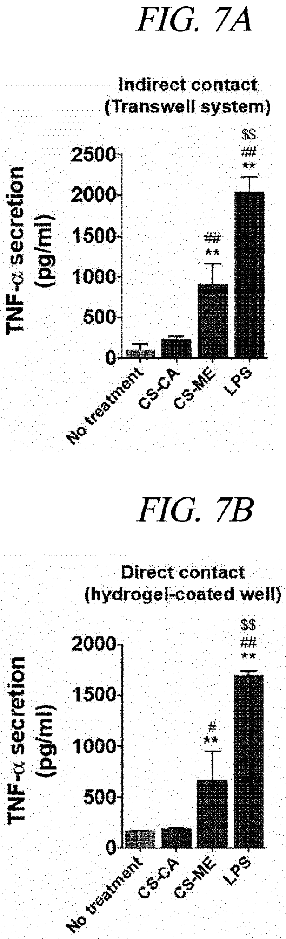

As a result, it was confirmed that the content of TNF-α secreted by the macrophages in co-culture with the CS-CA hydrogel was not high at a level almost equivalent to that of a group without any treatment regardless of the co-culture method, and the secretion amount of TNF-α significantly increased in co-culture with the CS-ME hydrogel **(****FIG. 7A** and **FIG. 7B****).**

It was confirmed from the above results that the CS-CA hydrogel shows a very low possibility of inducing an immune response compared to the conventional photo-crosslinked CS hydrogel.

### Example 7. Analysis on efficacy in promoting differentiation of stem cell in three-dimensional stem cell culture using chondroitin sulfate-catechol hydrogel (CS-CA hydrogel)

After three-dimensional culture of human adipose-derived stem cells (hADSC) in the CS-CA hydrogels, a test was conducted to differentiate the cells in a cartilage differentiation-inducing culture medium for 21 days and check the differentiation efficiency. Each test group was as follows: (1) Pellet group: a conventional culture method for culturing and differentiating cells in the form of pellets; (2) CS-CA group: a method for encapsulating stem cells in the CS-CA hydrogel for three-dimensional culture and differentiation; and (3) CS-CA w/dice group: a method for encapsulating stem cells with human-derived decellularized cartilage tissue dice in the CS-CA hydrogel for culture and differentiation in a three-dimensional environment similar to the actual cartilage tissue.

As for differentiation, the generation of glycosaminoglycan (GAG), which is a cartilage differentiation index, was quantified through DMB (1,9-dimethylmethylene blue) staining, followed by standardization to the total amount of DNA. As a result, it was confirmed that the amount of GAG was very low in the pellet group, whereas the amount of GAG significantly increased in the CS-CA hydrogel, which means that differentiation into chondrocytes is significantly enhanced **(****FIG. 8A****).** In particular, it was confirmed that the amount of GAG more significantly increased in the CS-CA w/dice group including the decellularized cartilage tissue dice in the CS-CA hydrogel than in the group using only the CS-CA hydrogel **(****FIG. 8A****).**

In addition, a test was conducted to check the expression levels of cartilage differentiation markers SOX9 and collagen type II (col2A1) through real-time qPCR. As a result, it was confirmed that the mRNA expression levels of SOX9 and collagen type II (col2A1) significantly increased in the CS-CA hydrogel group and the CS-CA w/dice group compared to the conventional pellet group **(****FIG. 8B****).**

Also, a test was conducted to check the expression level of collagen type II through immunofluorescence staining (cell nucleus: DAPI). As a result, it was confirmed that the expression level of collagen type II significantly increased in the CS-CA hydrogel group compared to the conventional pellet group. In particular, it was confirmed that the expression level of collagen type II further increased in the CS-CA w/dice group than in the CS-CA hydrogel group **(****FIG. 8C****).**

It was confirmed from the above results that the CS-CA hydrogel enables a three-dimensional culture of stem cells and has the effect of enhancing differentiation of stem cells.

### Example 8. Analysis on tissue implantation ability of chondroitin sulfate-catechol hydrogel (CS-CA Hydrogel)

A test was conducted to check autologous cartilage tissue implantation and the efficacy of cartilage formation in vivo in an animal model. In brief, a scaffold (hereinafter, referred to as "PCL scaffold") was prepared with a PCL (polycaprolactone) polymer produced by three-dimensional printing technique (in order to be used in the manufacture of an artificial ear graft by manufacturing an ear tissue scaffold in the future). Then, the cartilage was extracted from a rabbit's ear and mixed with the CS-CA hydrogel and applied onto the PCL scaffold to prepare a graft (FIG. 9).

The elastic modulus of the autologous cartilage graft implanted into the rabbit model was measured using a rheological analyzer. As a result, it was confirmed that due to the high mechanical strength of the PCL scaffold, the elastic modulus of the CS-CA hydrogel including the autologous cartilage tissue was significantly improved from 14 kPa to 1.1 mPa and was similar in value to the elastic modulus of the human cartilage tissue (0.2 mPa to 2.5 mPa) (FIG. 9).

The prepared graft and the PCL scaffold coated with the CS-CA hydrogel without the cartilage tissue were implanted subcutaneously in the same rabbit model, and the grafts were collected at 8, 16, 24 and 32 weeks after implantation, respectively, to check the efficacy of cartilage formation. As a result of visual observation of the graft extracted at 32 weeks after implantation, it was confirmed that the CS-CA hydrogel was biodegraded and the PCL scaffold was filled with the tissue in a form similar to the cartilage tissue **(****FIG. 10A**).

In particular, when histological analysis was conducted on the graft collected at 32 weeks after autologous cartilage tissue implantation, a significant amount of cartilage tissue was observed from the implanted three-dimensional PCL scaffold/CS-CA hydrogel graft including the autologous cartilage tissue **(****FIG. 11****).**

In addition, the amount of GAG formed in the graft was quantified through DMB staining, followed by standardization to the total amount of DNA. As a result, the amount of GAG significantly increased over time in the group in which the CS-CA hydrogel including the autologous cartilage tissue dice was implanted in the PCL scaffold compared to the group in which only the CS-CA hydrogel was implanted **(****FIG. 10B**).

In addition, a test was conducted to collect the cells in the graft, extract RNA and measure the mRNA expression levels of chondrocyte markers SOX9 and collagen type II (col2A1) through real-time qPCR. As a result, it was confirmed that the mRNA expression levels of SOX9 and collagen type II (col2A1) significantly increased in the group in which the CS-CA hydrogel including the autologous cartilage tissue dice was implanted in the PCL scaffold compared to the group in which only the CS-CA hydrogel was implanted, and the mRNA expression levels of SOX9 and collagen type II (col2A1) showed a tendency to increase over time **(****FIG. 10C**).

Also, after a rabbit's auricular cartilage tissue was implanted into a mouse model of auricular cartilage defect by using the CS-CA hydrogel or the conventional photo-crosslinked CS-ME hydrogel, a test was conducted to evaluate the efficiency of cartilage tissue implantation and the efficiency of tissue integration of the tissue adhesive CS-CA hydrogel. The mouse model of auricular cartilage defect was prepared by punching a 2 mm-hole through the pinna of the mouse with a biopsy punch.

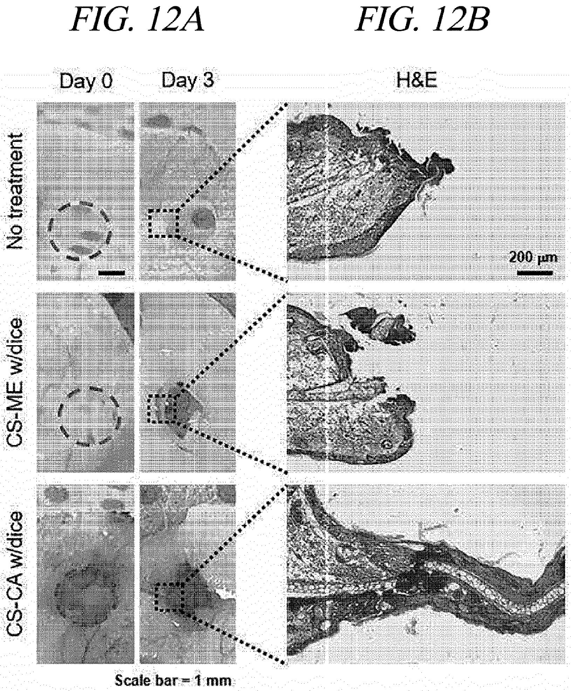

As a result, 3 days after cartilage implantation, the CS-ME hydrogel had no tissue adhesive properties and did not remain at the implanted site but was completely lost **(****FIG. 12A****).** However, the CS-CA hydrogel had excellent tissue adhesive properties and thus remained stably and was completely attached to the defective site of the tissue **(****FIG. 12A****).** In particular, as can be seen from the histological results, the CS-CA hydrogel enabled efficient integration of the implanted cartilage tissue through close connection with the surrounding tissues **(****FIG. 12B****).**

It was confirmed from the above results that the adhesive CS-CA hydrogel has the effect of promoting in vivo maintenance and engraftment of the cartilage tissue implanted for a long time and thus can be effectively used for autologous cartilage implantation, and has the effect of promoting cartilage formation.

### Example 9. Analysis on drug delivery ability of chondroitin sulfate-catechol hydrogel (CS-CA hydrogel)

A test was conducted to check a drug release behavior of the CS-CA hydrogel. In brief, TGF-β protein known to induce cartilage formation was loaded in the CS-CA hydrogel and a TGF-β release behavior was observed from a solution containing a PBS buffer or a chondroitinase at 37°C. The amount of TGF-β released was checked through ELISA analysis.

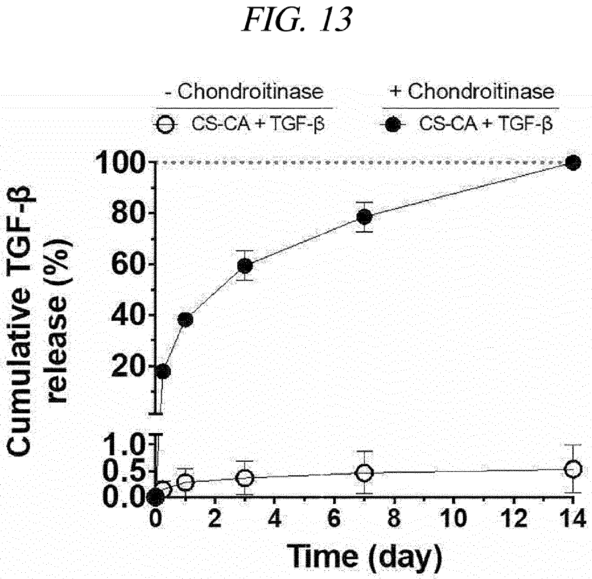

As a result, it was confirmed that when the chondroitinase was not treated, TGF-β was hardly released from the CS-CA hydrogel due to the strong binding of catechol and various nucleophilic functional groups of the protein, whereas when the chondroitinase was treated mimicking the in vivo human environment, TGF-β was gradually released from the CS-CA hydrogel for 2 weeks **(****FIG. 13****).**

It was confirmed from the above results that the CS-CA hydrogel can encapsulate a drug such as TGF-β and effectively deliver the drug for a long period of time at a tissue defective site in which activity of the chondroitinase increases.

### Example 10. Preparation of chondroitin sulfate-catechol hydrogel patch (CS-CA hydrogel patch) and analysis on drug delivery ability

In order to improve the convenience of application of the CS-CA hydrogel and to utilize it for various purposes, the CS-CA hydrogel was prepared in the form of a patch. A CS-CA hydrogel patch was prepared by thinly freezing the CS-CA solution and then lyophilizing it. A patch loaded with a drug was prepared by pre-dissolving a target drug before freezing the CS-CA solution and then lyophilizing them together. Also, the CS-CA hydrogel patch was crosslinked using an oxidizer (NaIO₄) spray to enable sustained release of the encapsulated drug.

In order to verify the applicability of the CS-CA hydrogel patch as a drug delivery system, a test was conducted to compare a growth factor protein release behavior between the following two groups: (1) Patch (CS-CA)-TGF- β group: a group prepared by encapsulating TGF-β protein in the prepared CA hydrogel patch; and (2) Patch (CS-CA)-TGF-β/FD group: a group prepared by previously mixing TGF-β protein with the CS-CA solution, lyophilizing the mixture in the form of a patch and crosslinking the CS-CA hydrogel patch.

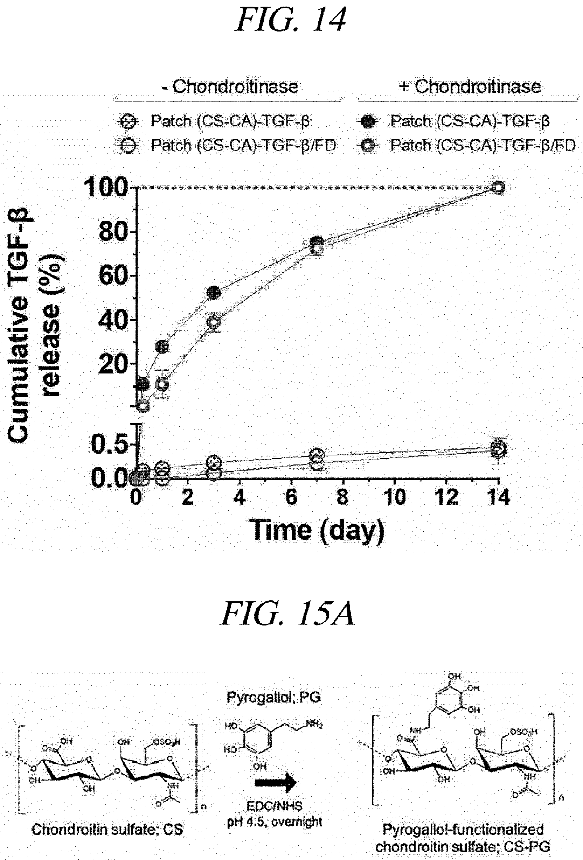

A TGF-β release behavior was observed from a solution containing a PBS buffer or a chondroitinase at 37°C, and the amount of TGF-β released was checked through ELISA analysis. As a result, it was confirmed that the patch (CS-CA)-TGF-β group in which TGF-β was encapsulated in the CS-CA hydrogel patch and the patch (CS-CA)-TGF-β/FD group in which TGF-β group were previously mixed with the CS-CA solution and then lyophilized showed similar drug release behaviors **(****FIG. 14****).** In particular, it was confirmed that when the chondroitinase was not treated, TGF-β was hardly released from the CS-CA hydrogel patch like the CS-CA hydrogel due to the strong binding of catechol and various nucleophilic functional groups of the protein, whereas when the chondroitinase was treated mimicking the in vivo human environment, TGF-β was gradually released from the CS-CA hydrogel for 2 weeks **(****FIG. 14****).**

It was confirmed from the above results that like the CS-CA hydrogel, the CS-CA hydrogel patch can also encapsulate a drug such as TGF-β and effectively deliver the drug for a long period of time at a tissue defective site in which activity of the chondroitinase increases.

### Example 11. Preparation of hydrogel based on chondroitin sulfate functionalized with pyrogallol

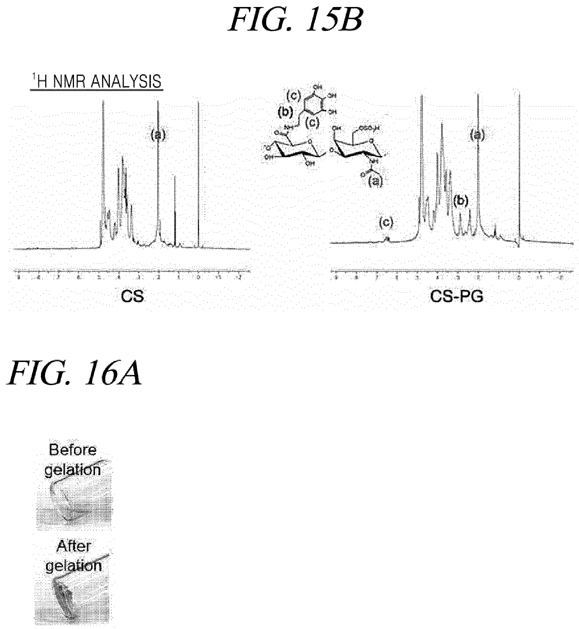

For synthesis of a derivative, chemical reaction between EDC (1-(3-dimethylaminopropyl)-3-ethylcarbodiimide hydrochloride (Thermo Fisher Scientific)) and NHS (N-hydroxysuccinimide (Sigma)) was applied to functionalize chondroitin sulfate (CS) with pyrogallol (PG) **(****FIG. 15A****).** CS:EDC:NHS:PG was prepared at 1:1:1:1 (molar ratio). Through ¹H-NMR analysis, it was confirmed from the synthesized derivative that chondroitin sulfate (CS) was successfully functionalized with pyrogallol **(****FIG. 15B****).**

Thereafter, a CS-PG solution was prepared using PBS and distilled water, and the CS-PG solution was treated with an oxidizer (sodium periodate (NaIO₄)) through a crosslinking reaction to prepare a pyrogallol-functionalized chondroitin sulfate-pyrogallol hydrogel (hereinafter referred to as "CS-PG hydrogel") **(****FIG. 16A****).** The CS-PG solution and the oxidizer (NaIO₄ solution, 4.5 mg/ml) were treated with 3:1 (volume ratio).

Pyrogallol further has a hydroxyl group (OH) in addition to the structure of catechol. Thus, it can crosslink a polymer through faster oxidation than catechol. Therefore, in an environment with high oxygen concentration, such as in the human body, natural oxidation can occur within a few minutes without treatment with an oxidizer. Accordingly, pyrogallol can be directly applied for crosslinking without treatment with an oxidizer. Since pyrogallol does not require additional treatment with an oxidizer for crosslinking, it can be considered as a very good material in terms of safety and biocompatibility.

### Example 12. Analysis on physicochemical properties of chondroitin sulfate-pyrogallol hydrogel (CS-PG hydrogel)

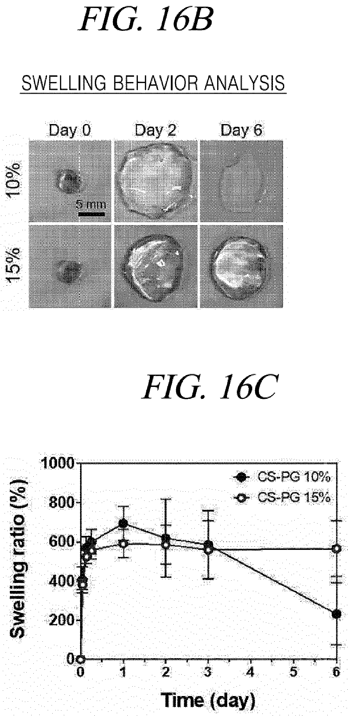

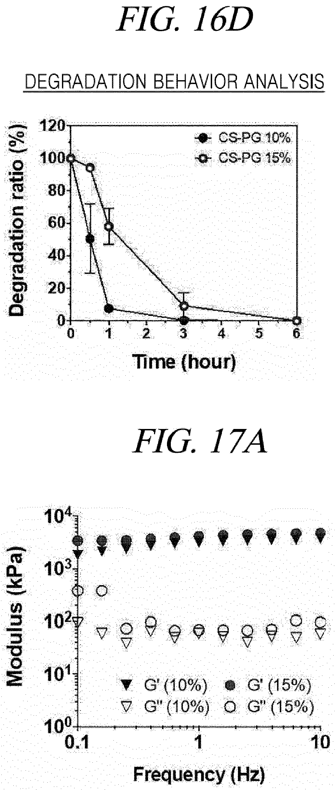

CS-PG hydrogels were prepared at two different concentrations (10 wt%, 15 wt%)) and tested to compare a swelling ratio and a degradation behavior. As a result, it was confirmed that the structure of the 15 wt% CS-PG hydrogel was maintained well in a swollen state without loss of mass under constant physiological conditions (37°C in a PBS buffer) for 6 days, whereas the structure of 10 wt% CS -PG hydrogel swelled until the 2nd day and then gradually collapsed and showed a large loss of mass. Thus, it was confirmed that the structure of the CS-PG hydrogel can be maintained well for a long period of time at a concentration of 15 wt% or more **(****FIG. 16B** and **FIG. 16C****).** Also, as a result of comparing the degradation behavior through treatment with a chondroitinase, it was confirmed that the degradation of the CS-PG hydrogel slowed down as the concentration of CS-PG increased **(****FIG. 16D****).**

### Example 13. Analysis on mechanical properties of chondroitin sulfate-pyrogallol hydrogel (CS-PG hydrogel)

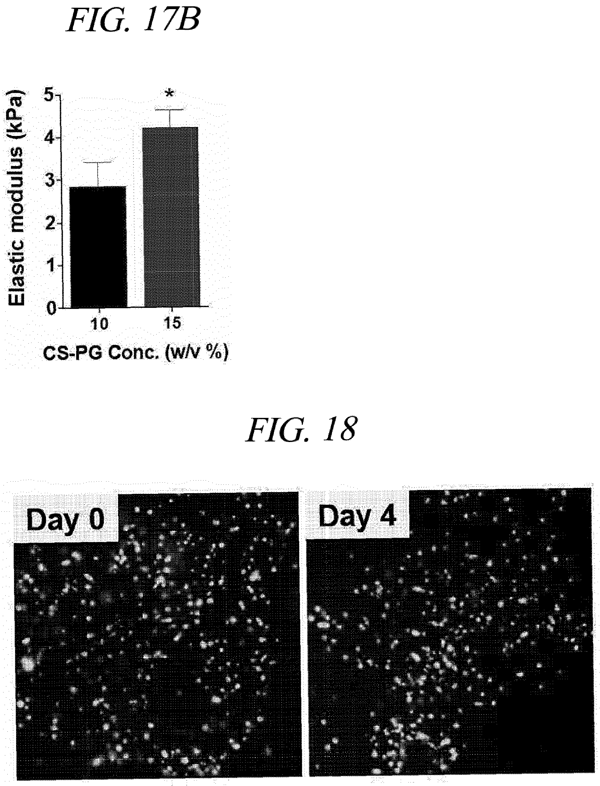

A test was conducted to measure the elastic modulus of the CS-PG hydrogel at two concentrations (10 wt%, 15 wt%) using a rheological analyzer. As a result, it was confirmed that the G' (storage modulus) value was higher than the G" (loss modulus) value at the both concentrations and thus a stable polymer network was formed in the hydrogel **(****FIG. 17A****).** Also, it was confirmed that as the concentration of CS-PG increased, the elastic modulus increased and the mechanical strength of the hydrogel was improved **(****FIG. 17B****).**

### Example 14. Analysis on cytotoxicity in three-dimensional stem cell culture using chondroitin sulfate-pyrogallol hydrogel (CS-PG hydrogel)

After three-dimensional culture of human adipose-derived stem cells (hADSC) in the CS-PG hydrogels, a test was conducted to analyze a cell survival rate. In brief, human adipose-derived stem cells were dispensed in each of the CS-PG hydrogels and cultured for 4 days, and then, the cell survival rate was analyzed through live/dead staining. As a result, it was confirmed that the human adipose-derived stem cells cultured in the CS-PG hydrogels showed a high survival rate and thus showed little cytotoxicity (FIG. 18). Accordingly, it was confirmed that the CS-PG hydrogels do not show cytotoxicity and thus have excellent biocompatibility.

## Claims

1. A hydrogel including chondroitin sulfate functionalized with a phenol derivative,
wherein the hydrogel has a structure represented by Chemical Formula 1: 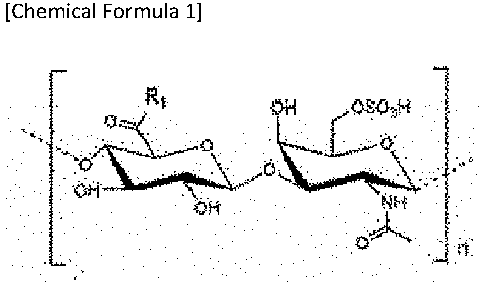
wherein in Chemical Formula 1, R₁ is a phenol derivative,
wherein the phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

2. The hydrogel of Claim 1,
wherein the hydrogel is prepared through oxidation of the functionalized phenol derivative.

3. The hydrogel of Claim 2,
wherein the oxidation is carried out by addition of an oxidizer or an enzyme.

4. The hydrogel of Claim 1,
wherein the hydrogel has an elastic modulus of from 0.1 kPa to 100 kPa at from 0.1 Hz to 10 Hz.

5. The hydrogel of Claim 1,
wherein the hydrogel has a tissue adhesive property.

6. The hydrogel of Claim 1,
wherein the hydrogel is biodegradable.

7. A composition for cell culture including a hydrogel of any one of Claim 1 to Claim 6.

8. The composition of Claim 7,
wherein the cell culture is a three-dimensional culture.

9. A composition for promoting cell differentiation including a hydrogel of any one of Claim 1 to Claim 6.

10. The composition of Claim 9,
wherein the cell is a stem cell.

11. The composition of Claim 10,
wherein the stem cell is selected from the group consisting of embryonic stem cells, fetal stem cells, induced pluripotent stem cells and adult stem cells.

12. The composition of Claim 9,
wherein the composition promotes differentiation into chondrocytes.

13. A composition for drug delivery including a hydrogel of any one of Claim 1 to Claim 6.

14. The composition of Claim 13,
wherein the drug is encapsulated or loaded in the hydrogel.

15. The composition of Claim 13,
wherein the drug is selected from the group consisting of an immune cell activator, an anticancer agent, a therapeutic antibody, an antibiotic, an antibacterial agent, an antiviral agent, an anti-inflammatory agent, a contrast medium, a protein drug, a growth factor, a cytokine, a peptide drug, a hair growth solution, an anesthetic and combinations thereof.

16. The composition of Claim 13,
wherein the composition is in the form of an adhesive patch or a film.

17. A composition for promoting tissue regeneration including a hydrogel of any one of Claim 1 to Claim 6.

18. A composition for tissue transplant including a hydrogel of any one of Claim 1 to Claim 6.

19. The composition of Claim 17 or Claim 18,
wherein the composition is in the form of an adhesive patch or a film.

20. The composition of Claim 17 or Claim 18,
wherein the tissue is selected from the group consisting of liver, heart, kidney, muscle, stomach, intestine, lung, bone, cartilage, blood vessel, bladder, skin, brain, fat, thyroid gland, salivary gland, oesophagus, pancreas, spinal cord, ligament, tendon, tooth and uterus.

21. A method of preparing a hydrogel based on chondroitin sulfate functionalized with a phenol derivative, comprising:
a process of functionalizing chondroitin sulfate with a phenol derivative,
wherein the hydrogel has a structure represented by Chemical Formula 1: 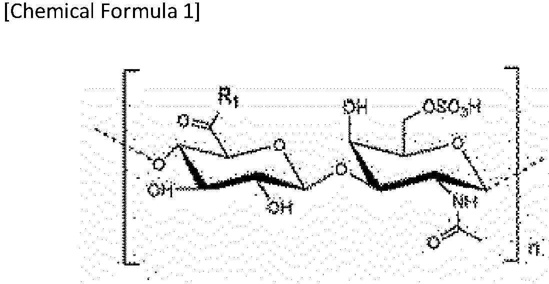
wherein in Chemical Formula 1, R₁ is a phenol derivative,
wherein the phenol derivative is a pyrogallol group derived from a pyrogallol-based compound selected from the group consisting of pyrogallol, 5-hydroxydopamine, tannic acid, gallic acid, epigallocatechin, epicatechin gallate, epigallocatechin gallate, 2,3,4-trihydroxybenzaldehyde, 2,3,4-trihydroxybenzoic acid, 3,4,5-trihydroxybenzaldehyde, 3,4,5-trihydroxybenzamide, 5-tert-butylpyrogallol and 5-methylpyrogallol.

22. The method of Claim 21, further comprising:
a process of oxidizing the functionalized phenol derivative.

## Patentansprüche

1. Hydrogel, welches mit einem Phenolderivat funktionalisiertes Chondroitinsulfat umfasst,
wobei das Hydrogel eine durch die Chemische Formel 1 dargestellte Struktur aufweist: 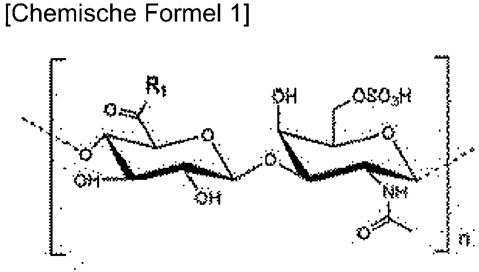
wobei in der Chemischen Formel 1 R₁ ein Phenolderivat ist,
wobei das Phenolderivat eine Pyrogallol-Gruppe ist, welche abgeleitet ist von einer Pyrogallol-basierten Verbindung, die ausgewählt ist aus der Gruppe bestehend aus Pyrogallol, 5-Hydroxydopamin, Tanninsäure, Gallussäure, Epigallocatechin, Epicatechingallat, Epigallocatechingallat, 2,3,4-Trihydroxybenzaldehyd, 2,3,4-Trihydroxybenzoesäure, 3,4,5-Trihydroxybenzaldehyd, 3,4,5-Trihydroxybenzamid, 5-tert-Butylpyrogallol und 5-Methylpyrogallol.

2. Hydrogel nach Anspruch 1,
wobei das Hydrogel durch Oxidation des funktionalisierten Phenolderivats hergestellt ist.

3. Hydrogel nach Anspruch 2,
wobei die Oxidation durch Zugabe eines Oxidationsmittels oder eines Enzyms ausgeführt ist.

4. Hydrogel nach Anspruch 1,
wobei das Hydrogel einen Elastizitätsmodul von 0,1 kPa bis 100 kPa bei 0,1 Hz bis 10 Hz aufweist.

5. Hydrogel nach Anspruch 1,
wobei das Hydrogel eine gewebeklebende Eigenschaft aufweist.

6. Hydrogel nach Anspruch 1,
wobei das Hydrogel bioabbaubar ist.

7. Zusammensetzung für Zellkultur, umfassend ein Hydrogel nach einem der Ansprüche 1 bis 6.

8. Zusammensetzung nach Anspruch 7,
wobei die Zellkultur eine dreidimensionale Kultur ist.

9. Zusammensetzung zur Förderung von Zelldifferenzierung, umfassend ein Hydrogel nach einem der Ansprüche 1 bis 6.

10. Zusammensetzung nach Anspruch 9,
wobei die Stammzelle eine Stammzelle ist.

11. Zusammensetzung nach Anspruch 10,
wobei die Stammzelle ausgewählt ist aus der Gruppe bestehend aus embryonalen Stammzellen, fetalen Stammzellen, induzierten pluripotenten Stammzellen und adulten Stammzellen.

12. Zusammensetzung nach Anspruch 9,
wobei die Zusammensetzung die Differenzierung zu Chondrozyten fördert.

13. Zusammensetzung für Arzneistoffzufuhr, umfassend ein Hydrogel nach einem der Ansprüche 1 bis 6.

14. Zusammensetzung nach Anspruch 13,
wobei der Arzneistoff in das Hydrogel eingekapselt oder geladen ist.

15. Zusammensetzung nach Anspruch 13,
wobei der Arzneistoff ausgewählt ist aus der Gruppe bestehend aus einem Immunzellenaktivator, einem Antikrebsmittel, einem therapeutischen Antikörper, einem Antibiotikum, einem antibakteriellen Mittel, einem antiviralen Mittel, einem entzündungshemmenden Mittel, einem Kontrastmittel, einem Protein-Arzneistoff, einem Wachstumsfaktor, einem Zytokin, einem Peptid-Arzneistoff, einer Haarwachstumslösung, einem Anästhetikum und Kombinationen davon.

16. Zusammensetzung nach Anspruch 13,
wobei die Zusammensetzung in der Form eines Klebepflasters oder eines Films ist.

17. Zusammensetzung zur Förderung von Geweberegenerierung, umfassend ein Hydrogel nach einem der Ansprüche 1 bis 6.

18. Zusammensetzung für Gewebetransplantat, umfassend ein Hydrogel nach einem der Ansprüche 1 bis 6.

19. Zusammensetzung nach Anspruch 17 oder 18,
wobei die Zusammensetzung in der Form eines Klebepflasters oder eines Films ist.

20. Zusammensetzung nach Anspruch 17 oder 18,
wobei das Gewebe ausgewählt ist aus der Gruppe bestehend aus Leber, Herz, Niere, Muskel, Magen, Darm, Lunge, Knochen, Knorpel, Blutgefäß, Blase, Haut, Gehirn, Fett, Schilddrüse, Speicheldrüse, Speiseröhre, Bauchspeicheldrüse, Rückenmark, Band, Sehne, Zahn und Gebärmutter.

21. Verfahren zur Herstellung eines Hydrogels, das auf mit einem Phenolderivat funktionalisierten Chondroitinsulfat basiert, umfassend:
einen Prozess des Funktionalisierens von Chondroitinsulfat mit einem Phenolderivat,
wobei das Hydrogel eine durch die Chemische Formel 1 dargestellte Struktur aufweist: 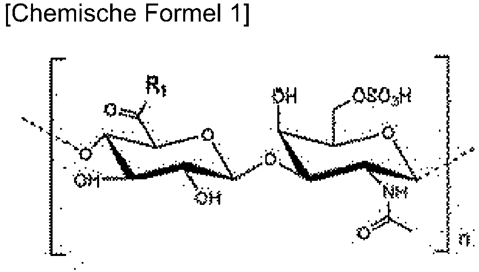
wobei in der Chemischen Formel 1 R₁ ein Phenolderivat ist,
wobei das Phenolderivat eine Pyrogallol-Gruppe ist, welche abgeleitet ist von einer Pyrogallol-basierten Verbindung, die ausgewählt ist aus der Gruppe bestehend aus Pyrogallol, 5-Hydroxydopamin, Tanninsäure, Gallussäure, Epigallocatechin, Epicatechingallat, Epigallocatechingallat, 2,3,4-Trihydroxybenzaldehyd, 2,3,4-Trihydroxybenzoesäure, 3,4,5-Trihydroxybenzaldehyd, 3,4,5-Trihydroxybenzamid, 5-tert-Butylpyrogallol und 5-Methylpyrogallol.

22. Verfahren nach Anspruch 21, welches zudem umfasst:
einen Prozess des Oxidierens des funktionalisierten Phenolderivats.

## Revendications

1. Hydrogel comportant du sulfate de chondroïtine fonctionnalisé avec un dérivé de phénol, l'hydrogel ayant une structure représentée par la formule chimique 1 : 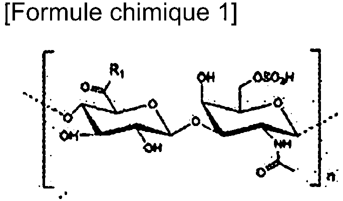
dans lequel, dans la formule chimique 1, R₁ est un dérivé de phénol,
dans lequel le dérivé de phénol est un groupement pyrogallolyle dérivé d'un composé à base de pyrogallol choisi dans le groupe constitué du pyrogallol, de la 5-hydroxydopamine, de l'acide tannique, de l'acide gallique, de l'épigallocatéchine, du gallate d'épicatéchine, du gallate d'épigallocatéchine, du 2,3,4-trihydroxy benzaldéhyde, de l'acide 2,3,4-trihydroxybenzoique, du 3,4,5-trihydroxybenzaldéhyde, du 3,4,5-trihydroxy benzamide, du 5-tert-butylpyrogallol, et du 5-méthylpyrogallol.

2. Hydrogel selon la revendication 1,
l'hydrogel étant préparé par oxydation du dérivé de phénol fonctionnalisé.

3. Hydrogel selon la revendication 2,
dans lequel l'oxydation est effectuée par addition d'un oxydant ou d'une enzyme.

4. Hydrogel selon la revendication 1,
l'hydrogel ayant un module élastique de 0,1 kPa à 100 kPa pour 0,1 Hz à 10 Hz.

5. Hydrogel selon la revendication 1,
l'hydrogel ayant une propriété adhésive vis à vis des tissus.

6. Hydrogel selon la revendication 1,
l'hydrogel étant biodégradable.

7. Composition pour culture cellulaire comportant un hydrogel selon l'une quelconque de la revendication 1 à la revendication 6.

8. Composition selon la revendication 7,
dans laquelle la culture cellulaire est une culture tridimensionnelle.

9. Composition pour promouvoir la différentiation cellulaire comportant un hydrogel selon l'une quelconque de la revendication 1 à la revendication 6.

10. Composition selon la revendication 9, dans laquelle la cellule est une cellule souche.

11. Composition selon la revendication 10,
dans laquelle la cellule souche est choisie dans le groupe constitué de cellules souches embryonnaires de cellules souches fœtales, de cellules souches induites pluripotentes, et de cellules souches adultes.

12. Composition selon la revendication 9,
la composition favorisant la différentiation en chondrocytes.

13. Composition pour l'administration d'un médicament comportant un hydrogel selon l'une quelconque de la revendication 1 à la revendication 6.

14. Composition selon la revendication 13,
dans laquelle le médicament est encapsulé ou chargé dans l'hydrogel.

15. Composition selon la revendication 13,
dans laquelle le médicament est choisi dans le groupe constitué d'un activateur de cellule immune, d'un agent anticancer, d'un anticorps thérapeutique, d'un antibiotique, d'un agent antibactérien, d'un agent antiviral, d'un agent anti-inflammatoire, d'un milieu de contraste, d'un médicament protéiné, d'un facteur de croissance, d'une cytokine, d'un médicament peptidique, d'une solution de croissance de cheveux, d'un anesthésique ou de combinaisons de ceux-ci.

16. Composition selon la revendication 13,
la composition étant sous la forme d'un patch adhésif ou d'un film.

17. Composition pour favoriser la régénération tissulaire comportant un hydrogel selon l'une quelconque de la revendication 1 à la revendication 6.

18. Composition pour implantation de tissu comportant un hydrogel selon l'une quelconque de la revendication 1 à la revendication 6.

19. Composition selon la revendication 17 ou la revendication 18,
la composition étant sous la forme d'un adhésif ou d'un film.

20. Composition selon la revendication 17 ou la revendication 18,
dans laquelle le tissu est choisi dans le groupe constitué de foie, de cœur, de rein, de muscle, d'estomac, d'intestin, de poumon, d'os, de cartilage, de vaisseau sanguin, de vessie, de peau, de cerveau, de graisse, de glande thyroïde, de glande salivaire, d'œsophage, de pancréas, de moelle épinière, de ligament, de tendon, de dent, et d'utérus.

21. Procédé de préparation d'un hydrogel à base de sulfate de chondroïtine fonctionnalisé avec un dérivé de phénol, comprenant :
un processus de fonctionnalisation du sulfate de chondroïtine avec un dérivé de phénol, dans lequel l'hydrogel possède une structure représentée par la formule chimique 1 : 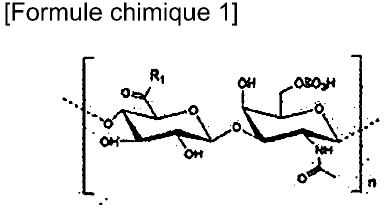
dans lequel, dans la formule chimique 1, R₁ est un dérivé de phénol,
dans lequel le dérivé de phénol est un groupement pyrogallolyle dérivé d'un composé à base de pyrogallol choisi dans le groupe constitué du pyrogallol, de la 5-hydroxydopamine, de l'acide tannique, de l'acide gallique, de l'épigallocatéchine, du gallate d'épicatéchine, du gallate d'épigallocatéchine, du 2,3,4-trihydroxybenzaldéhyde, de l'acide 2,3,4-trihydroxybenzoique, du 3,4,5-trihydroxybenzaldéhyde, du 3,4,5-trihydroxybenzamide, du 5-tert-butylpyrogallol, et du 5-méthylpyrogallol.

22. Procédé selon la revendication 21, comprenant en outre :
un processus d'oxydation du dérivé de phénol fonctionnalisé.
